(19) Europäisches Patentamt
European Patent Office
Office européen des brevets

(11) EP 3 048 433 A1

(12) EUROPEAN PATENT APPLICATION

(43) Date of publication:
27.07.2016 Bulletin 2016/30

(51) Int Cl.:
G01F 1/74 (2006.01)          G01F 15/08 (2006.01)
B67D 7/08 (2010.01)          A01J 5/01 (2006.01)

(21) Application number: 16152006.9

(22) Date of filing: 20.01.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 26.01.2015   DK 201500038

(71) Applicant: VM Tarm A/S
6880 Tarn (DK)

(72) Inventors:
• Berg-Konstmann, Kristian
6800 Varde (DK)
• Skovbjerg, Jan
6818 Årre (DK)
• Thomsen, Marius
6900 Skjern (DK)

(74) Representative: Nielsen, Charlotte
Tropa ApS
Aagade 97
8370 Hadsten (DK)

(54) TANKER AND METHOD APPLYING A DETECTION DEVICE

(57)    A tanker and a method applying a detection device (2) for detection of the gas content of a liquid-containing fluid is disclosed. The detection device (2) comprises a pressure difference inducing member (28), a first flow sensor (32) arranged at the first side of the pressure difference inducing member and a second flow sensor (32') arranged at the other side of the pressure difference inducing member. The detection device comprises a control unit configured to determine the gas content of the liquid-containing fluid by means of the flow measurements made by the flow sensors (32, 32').

The detection device (2) may comprise pressure sensors (30, 30') to measure the pressure on each side of the pressure difference inducing member (28). The pressure difference inducing member can be a valve or a pump or a pipe or a motor.

The tanker may be used for collection of milk (especially suitable for small quantities) or other gas containing liquids, where the gas content needs to be measured accurately.

Fig. 3

**Description**

**Field of invention**

The present invention generally relates to tanker and a method applying a device for detection of the gas content of a liquid. The present invention more particularly relates to tanker and a method applying a device for detecting the air content of a liquid delivered from a tank to a tanker or from a tank to a tank.

**Prior art**

From US 4,168,624, a method and an apparatus for determining the volume flowrates of a gas or a liquid forming a diphase mixture are known. The method and apparatus are applied in the oil industry in a pipe transporting the oil/gas mixture.

From US 4,050,896, a method and apparatus for the production of reaction mixtures from liquid reaction components for the production of foam material are known. In the method, one of the reaction components are charged with a gas, and the volumetric flowrate is a measurement for the gas charge. The method and apparatus are applied in the production of polyurethane foam.

From EP 1 686 355 A1, a method and a device for determining the mass flowrate of a gas/liquid mixture are known. The method and apparatus are applied in oil industry and used in connection with pumps or turbines.

From WO 91/15738 A1, a device and a method for measuring two-phase fluid flow are known. The device comprises first and second flow meters connected in series, thus providing signals indicative of first and second fluid flow, and further comprises a flow restrictor located between the first and the second flow meters for restricting the fluid flow, thereby obtaining signals indicative of the pressure in each of said flow meters. The device and method is applied in the oil industry as an inexpensive alternative to separating oil, gas and water to determine the content of the constituents.

From DE 10 2005 005 295 A1, a method and a device for determining the gas content of a liquid are known when transferring milk to or from a tanker. By the method, a pipe having a pump, which serves as a pressure difference inducing member, and pressure sensors are arranged on either side of the pressure difference inducing member for determining the mass flow through the pipe. The measurements are carried out continuously or intermittently.

From WO 2014/021884 A1, a method and a system for determining the characteristics of a multi-component fluid are known. The fluid comprises incompressible and compressible components. The system comprises first and second sensor assemblies separated within the fluid pipeline by a density adjuster. In an embodiment, the system may comprise first and second fluid meters, such as Coriolis flow meters.

During the process of filling a tanker with a fluid from a tank, the liquid is typically pumped into the tanker by means of a pump while measuring the flow of the fluid being pumped. However, since most liquids contain gas, it is essential to know the gas content of the liquid in order to establish an accurate measure of the amount of liquid received by the tanker.

Since the gas content of liquids such as milk varies considerably, it is necessary to know the gas content in order to be capable of detecting the liquid pumped into a tanker or a tank.

Accordingly, it would be desirable to have a simple and accurate method and device for detecting the gas content of a fluid.

Current tankers available on the market are not capable of providing liquid measurement with sufficient accuracy, unless the total quantity of liquid pumped into the tanker exceeds a certain minimum quantity (e.g. 300 litres). Accordingly, the prior art milk tankers are not capable of collecting milk from small farms.

Therefore, it would be desirable to have a method and a device adapted to collect small quantities of fluid in a manner in which the gas content can be measured with high accuracy.

Accordingly, it is an object of the present invention to provide a simple and accurate method applying a device for detecting the gas content of a fluid.

It is also an object of the present invention to provide a tanker and a method applying a device adapted to collect small quantities of fluid in a manner in which the gas content can be measured with high accuracy.

**Summary of the invention**

The object of the present invention can be achieved by a tanker as defined in claim 1 and by a method as defined in claim 7. Preferred embodiments are defined in the dependent sub claims, explained in the following description, and illustrated in the accompanying drawings.

The tanker is a tanker comprising a detection device for detection of the gas content of a liquid-containing fluid, wherein it comprises a gas separator arranged to degas the liquid-containing fluid before the liquid-containing fluid enters the detection device, wherein the device comprises a pressure difference inducing member, a first flow sensor arranged at a first side of the pressure difference inducing member and a second flow sensor arranged at the other side of the pressure difference inducing member, and a control unit configured to determine the gas content of the liquid-containing fluid by means of the flow measurements made by the flow sensors.

Hereby, it is possible to provide a device capable of detecting the gas content of a fluid in an easy an accurate manner. Applied in the tanker and the method according to the invention, it is possible to collect small quantities of fluid in a manner in which the gas content can be measured with high accuracy.

The detection device applied in the invention is a detection device for detection of the gas content of a liquid-containing fluid. The fluid may be milk, oil, gasoline or another liquid-containing gas. The detection device is configured to detect the gas content in a fluid comprising a liquid and gas mixture.

The device comprises a pressure difference inducing member that introduces a pressure difference. The pressure difference inducing member may increase the pressure or decrease the pressure. It is possible to apply different types of pressure difference inducing members including valves (e.g. check valves) and pumps (for increasing the pressure).

The detection device comprises a first flow sensor arranged at a first side of the pressure difference inducing member and a second flow sensor arranged at the other side of the pressure difference inducing member. The flow sensors may be of any suitable type and size. It is possible to apply two identical flow sensors; however, it is also possible to apply different types of flow sensors.

The detection device comprises a control unit configured to determine the gas content of the liquid-containing fluid by means of the flow measurements made by the flow sensors. The control unit may be of any suitable type and size. The control unit may be connected to the flow sensors by means of wires or by a wireless connection.

This makes it possible to provide a device capable of detecting the gas content of a fluid based on flow measurements and pressure information. The pressure may be detected by means of pressure sensors; however, the pressure information may be established in alternative ways. By way of example, it is possible to obtain pressure information based on level measurements of a tank or a supply pipe.

It may be an advantage that the detection device comprises a first pressure sensor arranged at a first side of the pressure difference inducing member and a second pressure sensor arranged at the other side of the pressure difference inducing member.

Hereby, it is possible to provide accurate measurements of the pressure at each side of the pressure difference inducing member at any desired time.

It may be beneficial that the detection device comprises one pressure sensor configured to detect the pressure difference across the pressure difference inducing member.

By having a detection device that comprises one pressure sensor configured to detect the pressure difference across the pressure difference inducing member, the pressure difference measurements can be conducted by means of one sensor.

It may be advantageous that the pressure difference inducing member is a valve. By using a valve as a pressure difference inducing member, a constant pressure difference may be provided across the pressure difference inducing member. Accordingly, a reliable and robust pressure difference inducing member can be provided in a simple way. The valve may be an electronically or pneumatically controlled valve. Hereby, the pressure difference introduced by the valve may be controlled.

If the pressure difference inducing member is a constant pressure valve, the pressure at each side of the pressure difference inducing member may be determined by measuring the pressure at one of the sides of the pressure difference inducing member.

It may be advantageous that the pressure difference inducing member is a pump. The pump may, at the same time, provide the pressure required for the fluid to flow through the detection device. Moreover, the pump may be configured to be regulated (e.g. by means of a control box regulating the speed of the pump) if the pressure should be changed.

It may be beneficial that the sensors are configured to provide continuous monitoring measurements. By applying sensors that are configured to provide continuous monitoring measurements, the detection device will be updated constantly with gas content data or data required for calculating the gas content. This means that the detection device will be capable of detecting if and when changes occur, e.g. when milk with high air content is pumped into a tanker.

It may be advantageous that the sensors are configured to provide intermittent measurements. The use of sensors that are configured to provide intermittent measurements may be sufficient in some circumstances. They may be cheaper and require less data to be stored.

By way of example, it may be possible to provide measurements at least every second, every 10 seconds, twice every minute or once every minute.

It may be an advantage that the detection device is configured to detect the gas content, $V_{air\ 1}$, in the fluid entering the detection device by means of the following formula:

$$V_{air1} = \frac{V_2 - V_1}{\frac{P_1}{P_2} - 1}$$

In the formula, $V_1$ is the volume of fluid at the first side of the pressure difference inducing member, $V_2$ is the volume of fluid at the other side of the pressure difference inducing member, $P_1$ is the pressure at the first side of the pressure difference inducing member and $P_2$ is the pressure at the other side of the pressure difference inducing member.

It may be beneficial that the detection device comprises a number of temperature sensors configured to detect the temperature of the fluid at both the low pressure side of the pressure difference inducing member and at the high pressure side of the pressure difference inducing member.

It may be an advantage that the detection device comprises pressure sensors configured to measure the absolute pressure at each side of the pressure difference inducing member.

Alternatively, the detection device may comprise pressure sensors configured to measure the absolute pressure at one side of the pressure difference inducing member as well as the pressure difference across the pressure difference inducing member.

It may be an advantage that the detection device comprises an inlet pipe and an outlet pipe.

The detection device is preferably integrated in a tanker such as a feed tanker, a liquid manure trailer, a chemical trailer, a milk tanker, a vacuum tanker or a beer tanker.

The detection device is preferably integrated in a milk tanker.

It may be advantageous that the pressure difference inducing member is formed as a spring-loaded check valve arranged between a first pressure sensor which is arranged at the low pressure side of the check valve and a second flow sensor which is arranged at the high pressure side of the check valve. Such a construction is simple and reliable.

It may be an advantage that the pressure difference inducing member is configured to introduce a pressure drop causing the second pressure sensor arranged at the low pressure side of the pressure difference inducing member to detect a pressure that is lower than the pressure measured by means of the first pressure sensor.

It may be beneficial that the tanker comprises a container and an air evacuation pipe being in fluid communication with the container, preferably with the upper portion of the container of the tanker, wherein the evacuation pipe is connected to the gas separator through a connection pipe. Hereby, air/gas from the container of the tanker can be evacuated through the gas separator.

It may be advantageous that the detection device comprises a gas separator shaped as a tank provided with a dipstick having a stick member. Hereby, it is possible to detect the fluid level of the gas separator in an easy manner.

The method according to the invention is a method for detection of the gas content of a liquid-containing fluid in a tanker, which method comprises the following steps:

- passing the liquid-containing fluid through a gas separator before the gas content of the liquid-containing fluid is detected;
- establishing a pressure difference across a pressure difference inducing member;
- detecting a first flow at a first side of the pressure difference inducing member by means of a first flow sensor arranged at the first side of the pressure difference inducing member;

- detecting a second flow at the other side of the pressure difference inducing member by means of a second flow sensor arranged at the other side of the pressure difference inducing member;
- determining the gas content of the liquid-containing fluid by means of the flow measurements made by the flow sensors.

The method according to the invention allows for easy and accurate measurement of the gas content of a liquid-containing fluid.

Determining the gas content of the liquid-containing fluid by means of the flow measurements made by the flow sensors may include applying pressure information. Pressure information may be obtained by any suitable means.

It may be beneficial that the method comprises the step of measuring the pressure difference across the pressure difference inducing member by means of one or more pressure sensors. If the absolute pressure is measured (or known) at one side of the pressure difference inducing member, this measurement together with the pressure difference across the pressure difference inducing member will be sufficient to calculate the gas content of the fluid.

By measuring the pressure difference across the pressure difference inducing member by means of one or more pressure sensors, it is possible to obtain an accurate measurement of the pressure difference across the pressure difference inducing member.

It may be an advantage to provide a first pressure measurement at a first side of the pressure difference inducing member and a second pressure measurement at the other side of the pressure difference inducing member.

Hereby, it is possible to obtain accurate pressure measurements conducted at each side of the pressure difference inducing member.

The pressure difference measurements may be carried out by means of one pressure sensor configured to detect the pressure difference across the pressure difference inducing member. Accordingly, the pressure difference measurements can be conducted by means of only one sensor. This approach requires that the absolute pressure is known at one side of the pressure difference inducing member.

It may be beneficial that the method comprises the step of measuring the pressure at each side of the pressure difference inducing member by means of one or more pressure sensors.

It may be an advantage that the pressure difference inducing member is a valve or a pump or a pipe or a motor.

By applying a valve as a pressure difference inducing member, it is possible to provide a very simple and reliable method.

Likewise, the use of a pump as a pressure difference inducing member may be advantageous as the pump may be applied to generate the required pressure for transportation of the fluid being measured.

However, it may be possible to apply a pipe having a known geometry to generate a pressure difference.

It may be beneficial that the measurements are continuous measurements or intermittent measurements.

It may be advantageous that the pressure difference inducing member is a constant pressure valve.

In one embodiment, the pressure difference inducing member is an air-operated constant pressure valve.

The method according to the invention may preferably be applied in tankers, such as liquid manure trailers, chemical trailers, milk tankers, vacuum tankers and beer tankers.

It may be an advantage that the method determines the gas content, $V_{air\,1}$, in the fluid by using the following formula:

$$V_{air\,1} = \frac{V_2 - V_1}{\frac{P_1}{P_2} - 1}$$

Where $V_1$ is the volume of fluid at the first side of the pressure difference inducing member, $V_2$ is the volume of fluid at the other side of the pressure difference inducing member, $P_1$ is the pressure at the first side of the pressure difference inducing member, and $P_2$ is the pressure at the other side of the pressure difference inducing member.

It may be beneficial that the method comprises the step of detecting the temperature of the fluid at both the low pressure side of the pressure difference inducing member and at the high pressure side of the pressure difference inducing member.

In case of detection of a temperature difference between the high pressure side and the low pressure side of the pressure difference inducing member, the method may take this temperature difference into consideration when calculating the gas content, as $PV=nRT$, where P is the absolute pressure of the gas, V is the volume of the gas, n is the amount of substance of gas (measured in moles), T is the absolute temperature of the gas, and R is the ideal gas constant.

**Description of the Drawings**

The invention will become more fully understood from the detailed description given herein below. The accompanying drawings are given by way of illustration only, and thus, they are not limitative of the present invention. In the accompanying drawings:

Fig. 1 A)   shows a schematic view of a first embodiment of a detection device applied according to the invention;
Fig. 1 B)   shows a schematic view of a second embodiment of a detection device applied according to the invention;
Fig. 2 A)   shows a schematic view of a third embodiment of a detection device applied according to the invention;
Fig. 2 B)   shows a schematic view of a first embodiment of a detection device applied according to the invention;
Fig. 3   shows a schematic view of a detection device applied according to the invention integrated in a tanker;
Fig. 4   schematically illustrates a detection device applied according to the invention, in which the liquid and gas portions of a fluid are graphically shown at both the low and high pressure side of a pressure difference inducing member;
Fig. 5 A)   shows a schematic view of a detection device applied according to the invention;
Fig. 5 B)   shows a schematic view of the detection device shown in Fig. 5 A), where the flow direction has been changed,
Fig. 6 A)   shows a schematic view of a detection device without pressure sensors and

Fig. 6 B)     shows a schematic view of a detection device with a pressure difference sensor.

**Detailed description of the invention**

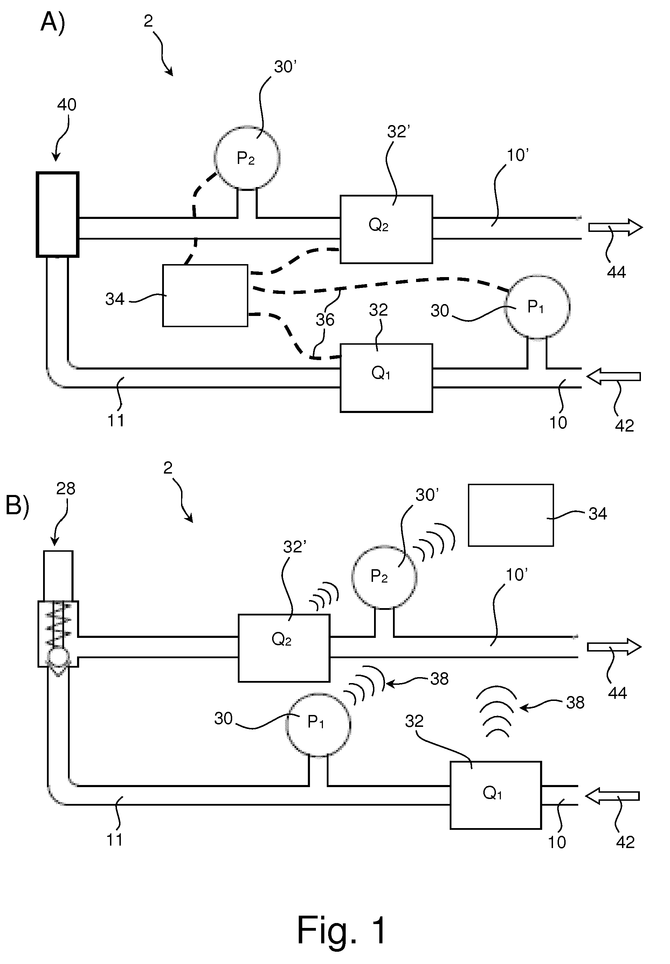

Referring now in detail to the drawings for the purpose of illustrating preferred embodiments of the present invention, different embodiments of a detection device applied in the invention are illustrated in Fig. 1.

Fig. 1 A) illustrates a schematic view of a first embodiment of a detection device 2 applied in the invention. The detection device 2 is configured to detect the gas content of a liquid flowing through the pipe system comprising the pipes 10, 10', 11. The liquid may be e.g. oil, gasoline or milk. However, the detection device 2 may be applied to detect the gas content of other types of liquids as well.

The detection device 2 comprises a pipe system comprising a plurality of pipes 10, 10', 11. The detection device 2 further comprises a pressure difference inducing member 40. The detection device 2 is adapted to receive a fluid (the liquid and gas mixture) through the pipe 10 (indicated by a first arrow 42). The fluid may be milk.

The pressure $P_1$ of the fluid (the liquid and gas mixture) entering the pipe 10 is detected by the pressure sensor 30. A first flow sensor 32 is arranged between the first pressure sensor 30 and the pressure difference inducing member 40. The first flow sensor 32 is adapted to detect the flow $Q_1$ of the fluid (the liquid and gas mixture) entering the pipe 10.

The pressure difference inducing member 40 introduces a pressure drop causing the second pressure sensor 30' arranged at the low pressure side of the pressure difference inducing member 40 to detect a pressure $P_2$ that is lower than the pressure $P_1$ measured by means of the first pressure sensor 30. A second flow sensor 32' arranged next to the second pressure sensor 30' is adapted to detect the flow $Q_2$ fluid flowing through the pipe 10' of the detection device 2. The fluid in the detection device 2 leaves the detection device 2 through the pipe 10' (indicated by a second arrow 44).

The flow sensors 30, 30' as well as the pressure sensors 32, 32' are electrically connected to a control unit 34 by means of wires 36. As the control unit 34 receives pressure and flow measurements from the flow sensors 30, 30' and the pressure sensors 32, 32', the control unit 34 can determine the gas percentage of the fluid.

As the liquid portion of the fluid is incompressible, the flow $Q_{Liquid\ 1}$ of the liquid entering the pipe 10 equals the flow $Q_{Liquid\ 2}$ of the liquid leaving the pipe 10'. During a given time period, t, the volume of liquid $V_{Liquid\ 1}$ flowing through the first pipe 10 equals the volume of liquid $V_{Liquid\ 2}$ flowing through the second pipe 10'. This can be expressed in the following way:

$$(1) \qquad Q_{Liquid\ 1} = Q_{Liquid\ 2}$$

$$(2) \qquad tQ_{Liquid\ 1} = V_{Liquid\ 1} = V_{Liquid\ 2} = tQ_{Liquid\ 2}$$

As an approximation, one may assume that the air content in the fluid acts as an ideal gas. Accordingly, the ideal gas equation can be applied:

$$(3) \qquad PV = nRT,$$

P is the absolute pressure of the gas, V is the volume of the gas, n is the amount of substance of gas (measured in moles), T is the absolute temperature of the gas, and R is the ideal gas constant.

As T, R and n are constant during application of the device 2, it follows that the product between the pressure P and the volume V is constant. This can be expressed in the following way:

$$(4) \qquad PV = Constant$$

At the low pressure side of the pressure difference inducing member 40 it follows that:

$$(5) \qquad P_1 V_{air\ 1} = C,$$

where $P_1$ is the pressure, C is the constant, and $V_{air\ 1}$ is the volume of the air.

At the high pressure side of the pressure difference inducing member 40, it follows that:

$$(6) \qquad\qquad P_2V_{air\,2} = C,$$

where $P_2$ is the pressure, C is the constant, and $V_{air\,2}$ is the volume of the air.

Combining (4) and (5), it follows that:

$$(7) \qquad\qquad P_1V_{air\,1} = P_2V_{air\,2}$$

or expressed differently:

$$(8) \qquad\qquad V_{air\,1} = \frac{P_2}{P_1}V_{air\,2}$$

Thus, the air flow $Q_{air\,1}$ through the first flow sensor 32 is given by:

$$(10) \qquad\qquad Q_{air\,1} = \frac{Q_2 - Q_1}{\frac{P_1}{P_2} - 1}$$

and

$$(11) \qquad\qquad V_{air\,1} = \frac{V_2 - V_1}{\frac{P_1}{P_2} - 1}$$

Hence, the detection device 2 can be used to detect the air content in the fluid entering the pipe 10 of the detection device 2.

Fig. 1 B) illustrates a schematic view of a second embodiment of a detection device 2 applied in the invention. The detection device 2 basically corresponds to the one shown in Fig. 1A. However, the pressure difference inducing member is formed as a spring-loaded check valve 28 arranged between a first pressure sensor 30 which is arranged at the low pressure side of the check valve 28 and a second flow sensor 32' which is arranged at the high pressure side of the check valve 28.

A second pressure sensor 30' is arranged next to the second flow sensor 32', whereas a first flow sensor 32 is arranged next to the first pressure sensor 30 near the inlet pipe 10 of the detection device 2.

Furthermore, each of the pressure sensors 30, 30' and the flow sensors 32, 32' are equipped with a wireless transmitter configured to communicate wirelessly with a control unit 34 arranged next to the outlet pipe 10' of the detection device 2.

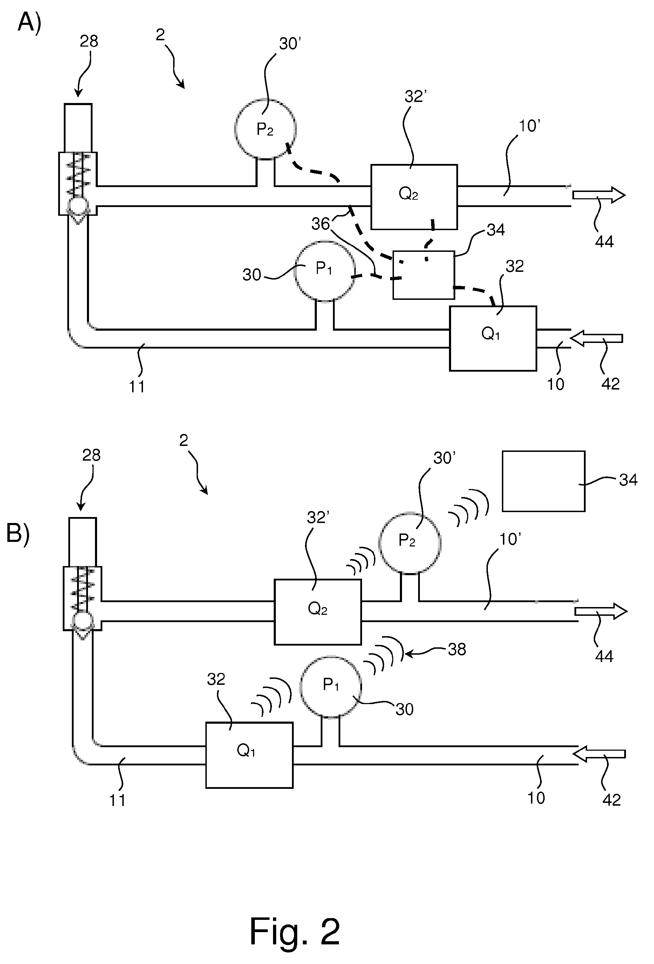

Fig. 2 A) illustrates a schematic view of a third embodiment of a detection device 2 applied in the invention. The detection device 2 comprises a first pressure sensor 30 arranged at the low pressure side of a check valve 28 introducing a fixed and constant pressure difference of e.g. 1 bar. The first pressure sensor 30 detects a first pressure $P_1$. The detection device 2 comprises a second pressure sensor 30' arranged at the low pressure side of a check valve 28. The second pressure sensor 30' detects a pressure $P_2$ that is significantly lower than the first pressure $P_1$ due to the pressure drop $P_1$-$P_2$ introduced by the check valve 28.

The detection device 2 comprises a first flow sensor 32 detecting a first flow $Q_1$ of the fluid entering the detection device 2 through the inlet pipe 10. The detection device 2 further comprises a second flow sensor 32' detecting a second flow $Q_2$ of the fluid flowing through the outlet pipe 10' of the detection device 2. The second flow sensor 32' is arranged between the second pressure sensor 30' and the outlet pipe 10'.

The detection device 2 comprises a control unit 34 electrically connected to the first flow sensor 32, the second flow sensor 32', the first pressure sensor 30 and the second pressure sensor 30'. The control unit 34 may be configured to detect the air content in the fluid entering the pipe 10 of the detection device 2 by using the method explained with reference to Fig. 1 A).

Fig. 2 B) schematically illustrates another embodiment of a detection device 2 applied in the invention. The detection device 2 comprises a first flow sensor 32 arranged at the low pressure side of a check valve 28. The first flow sensor 32 detects a first flow $Q_1$. The detection device 2 comprises a second flow sensor 32' arranged at the high pressure side of a check valve 28. The second flow sensor 32' detects a flow $Q_2$

The detection device 2 comprises a first pressure sensor 30 detecting a first pressure $P_1$ of the fluid entering the detection device 2 through the inlet pipe 10. The detection device 2 further comprises a second pressure sensor 30' detecting a second pressure $P_2$ of the fluid flowing through the outlet pipe 10' of the detection device 2. The second pressure sensor 30' is arranged between the second flow sensor 32' and the outlet pipe 10'.

The second pressure sensor 30' detects a pressure $P_2$ that is significantly lower than the first pressure $P_1$ due to the pressure drop $P_1$-$P_2$ introduced by the check valve 28.

The detection device 2 comprises a control unit 34 wirelessly connected to the first flow sensor 32, the second flow sensor 32', the first pressure sensor 30 and the second pressure sensor 30'.

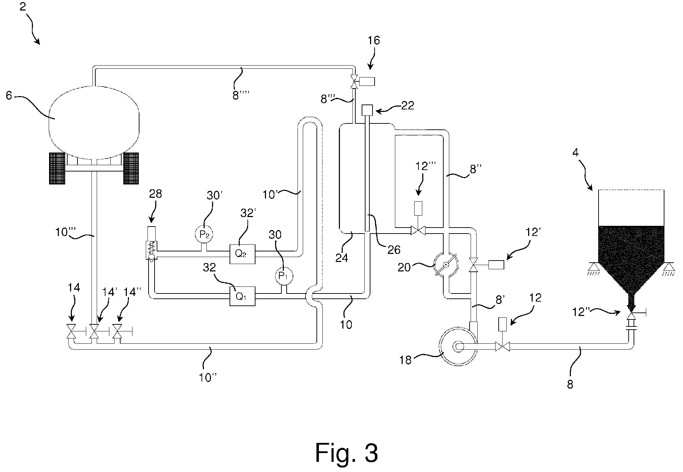

Fig. 3 illustrates a schematic view of a detection device 2 applied in a tanker according to the invention. The tanker comprises a container 6 configured to be brought into fluid communication with an (external) inlet tank 4. The inlet tank 4 may be e.g. a milk tank applied for milk storage at a farm.

The inlet tank 4 comprises a pipe 8 and a control valve 12" configured to be fully or partially opened or closed in order to adjust the fluid flow.

At the end of the pipe 8, a control valve 12 is provided. The pipe 8 is connected to a main pump 18 adapted to pump fluid from the inlet tank 4 into and through the detection device 2.

The detection device 2 comprises a gas separator 24 shaped as a tank provided with a dipstick 22 having a stick member 26. A pipe 8' connects the main pump 18 to a feeder pump 20 and a valve 12' controlling the degree of access to a bypass pipe (bypassing the feeder pump 18). An outlet pipe is provided at the bottom portion of the gas separator 24, and a valve 12''' is provided in order to close and open the outlet pipe (for the purpose of emptying the gas separator 24). A pipe 8" is connected to the upper portion of the gas separator 24.

An inlet pipe 10 connects the bottom portion of the gas separator 24 with a first pressure sensor 30 of the detection device 2. A first flow sensor 32 is provided next to the first pressure sensor 30. The first pressure sensor 30 detects a first pressure $P_1$, whereas the first flow sensor 32 detects a first flow $Q_1$.

A second pressure sensor 30' configured to detect a second pressure $P_2$ is arranged in fluid communication with the first flow sensor 32, and a check valve 28 is provided between the first flow sensor 32 and the second pressure sensor 30'. A second flow sensor 32' adapted to measure a second flow $Q_2$ is arranged next to the second pressure sensor 30'. The sensor configuration corresponds to the one illustrated in Fig. 1A.

The second flow sensor 32' is provided at the outlet pipe 10' which is in fluid communication with a connection pipe 10" provided with three end valves 14, 14', 14" each configured to be connected to an inlet pipe 10''' which is connected to a container 6 of the tanker.

An air evacuation pipe 8'''' is provided at the upper portion of the container 6 of the tanker. The evacuation pipe 8'''' is connected to the gas separator 24 through a connection pipe 8'''. A valve 16 connects the evacuation pipe 8'''' and the connection pipe 8'''. The detection device 2 may be shaped to evacuate air differently. The air may be evacuated to the atmosphere or into a vessel (e.g. a vessel for foam).

By using the detection device 2 in accordance with the invention, it is possible to detect the air content of the fluid entering the inlet pipe 10 in an easy and accurate manner. Thus, by means of the present invention, it is possible to provide a liquid measurement with sufficiently high accuracy.

The detection device is adapted to collect small quantities of fluid in a manner in which the gas content can be measured with high accuracy.

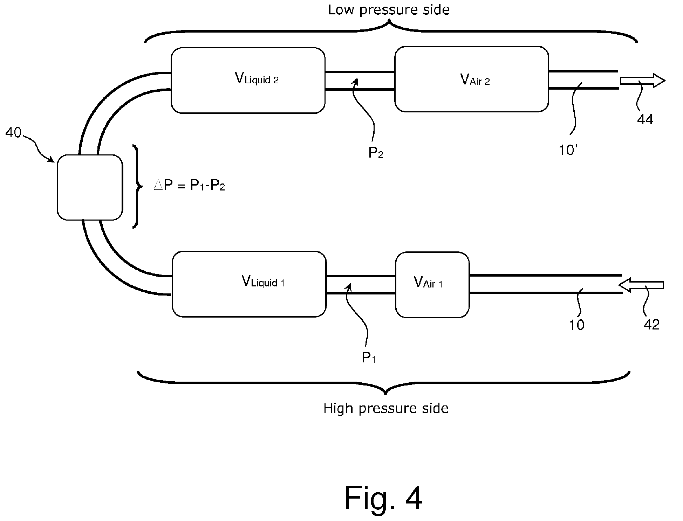

Fig. 4 schematically illustrates a detection device according to the invention. The liquid portions $V_{liquid\ 1}$, $V_{liquid\ 2}$ and the gas portions $V_{air\ 1}$, $V_{air\ 2}$ of a fluid being measured by means of the detection device are graphically shown in Fig. 4. It can be seen that the gas portion volume $V_{air\ 1}$ is significantly lower at the high pressure side of the pressure difference inducing member 40 than the gas portion volume $V_{air\ 2}$ at the low pressure side of the pressure difference inducing member 40.

The pressure drop across the pressure difference inducing member 40 causes an increasement of the gas portion volume $V_{air\ 2}$ as illustrated in Fig. 4. Due to the fact that the liquid of the fluid is incompressible, the volume of the liquid portions $V_{liquid\ 1}$, $V_{liquid\ 2}$ remains constant.

Accordingly, the flow of the liquid entering the inlet pipe 10 equals the flow of the liquid leaving the detection device through its outlet pipe 10' (indicated by an arrow 44).

By applying a predefined (preferably constant) pressure drop across the pressure difference inducing member 40, it is only necessary to establish (e.g. by a direct or an indirect measurement) the absolute pressure at one side of the pressure difference inducing member 40 and to measure the flow of the fluid at each side of the pressure difference inducing member 40 in order to detect the volume of the gas portion, $V_{air\ 1}$, of the fluid entering the inlet pipe 10 (indicated by the arrow 42).

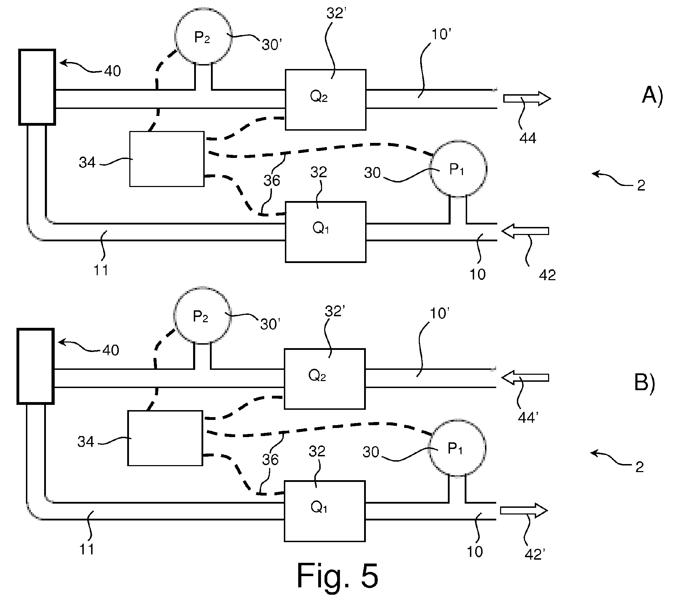

Fig. 5 A) illustrates a detection device 2 corresponding to the one shown in Fig. 1A. The detection device 2 is configured to detect the air portion of a liquid- and air-containing fluid entering the inlet pipe 10 of the detection device 2 and leaving the detection device 2 through the outlet pipe 10'. The flow direction of the fluid is indicated by means of a first arrow 42 and a second arrow 44.

Fig. 5 B) illustrates a similar detection device 2. The fluid, however, enters the inlet pipe 10' of the detection device 2 and leaves the detection device 2 through the outlet pipe 10. Accordingly, the flow direction has changed when compared to Fig. 5 A). The flow direction of the fluid is indicated by means of a first arrow 42' and a second arrow 44'.

The detection device 2 comprises a pressure difference inducing member 40 , pressure sensors 30, 30', flow sensors 32, 32' as well as a control unit 34 which is electrically connected to the sensors 30, 30', 32, 32'.

The pressure sensors 30, 30' may be omitted if the pressure difference inducing member causes a predefined, constant pressure drop (e.g. in the range of 0.5-2 bar), and the absolute pressure is known (e.g. measured) at one side of the pressure difference inducing member 40. If fluid is delivered from a storage tank with a constant and well-defined pressure, pressure measurements may be omitted if the pressure difference inducing member causes a predefined and constant pressure drop.

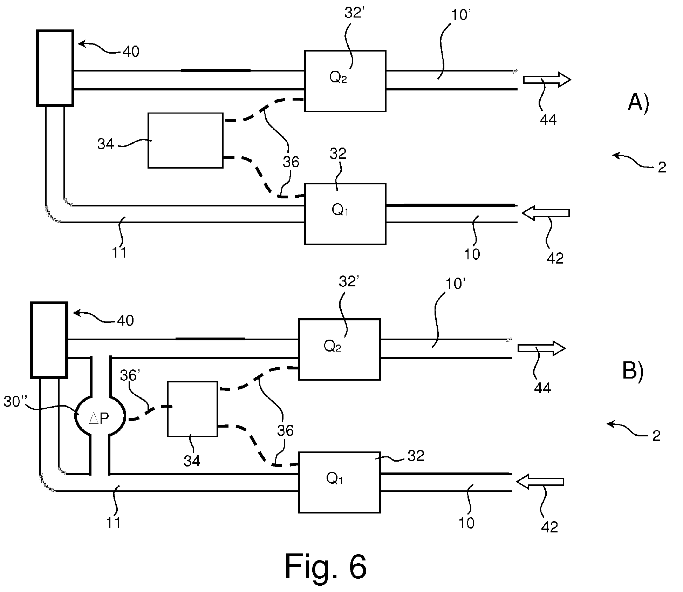

Fig. 6 A) illustrates a schematic view of a detection device 2 without pressure sensors. The detection device 2 comprises an inlet pipe 10 through which a fluid enters the detection device 2. The direction of the fluid is indicated by an arrow 42.

The detection device 2 comprises a first flow sensor 32 configured to measure the flow $Q_1$ of the fluid flowing through the inlet pipe 10. The fluid flows further through a connection pipe 11 that is in fluid communication with a pressure difference inducing member 40 (e.g. a valve) that introduces a pressure drop. At the low pressure side of the pressure difference inducing member 40, the fluid flows through an outlet pipe 10' provided with a second flow sensor 32' which is configured to measure the flow of the fluid at the low pressure side of the pressure difference inducing member 40.

A control unit 34 is electrically connected to the first flow sensor 32 and to the second flow sensor by means of wires 36. As the pressure difference inducing member 40 introduces a constant well-defined pressure drop, it is not necessary to measure the pressure difference across it. Accordingly, a very simple detection device can be provided.

It would however, be necessary to know the absolute pressure at one side of the pressure difference inducing member 40. This may be measured by means of a sensor (not shown) or be established otherwise.

Pressure information may be provided by external means (e.g. a level sensor from a tank). In some situations, the pressure may be known (e.g. if the pressure is predefined from the fluid source).

Fig. 6 B) illustrates a schematic view of a detection device 2 applied in the invention. The detection device 2 is equipped with a difference pressure sensor 30" configured to measure the pressure difference ∆P across the pressure difference inducing member 40. The difference pressure sensor 30" is connected to the pipes 10', 11.

The remaining structure of the detection device 2 illustrated in Fig. 6 B) corresponds to the detection device 2 illustrated in Fig. 6 A).

**List of reference numerals**

| | |
|---|---|
| 2 | Detection device |
| 4 | Inlet tank |
| 6 | Container of a tanker |
| 8, 8', 8", 8''', 8"" | Pipe |
| 10, 10', 10", 10''', 11 | Pipe |
| 12, 12', 12", 12''' | Valve |
| 14, 14', 14", 46 | Valve |
| 18 | Main pump |
| 20 | Feeder pump |
| 22 | Dipstick |
| 24 | Gas separator |
| 26 | Stick member |
| 28 | Valve |
| 30, 30', 30" | Pressure sensor (manometer) |
| 32, 32' | Flow sensor |
| 34 | Control unit |
| 36, 36' | Wire |
| 38 | Wireless signal |
| 40 | Pressure difference inducing member |

| 42, 42', 44, 44' | Arrow |
| $P_1$, $P_2$ | Pressure |
| t | Time |
| $\Delta P$ | Pressure difference |
| $Q_1$, $Q_2$, $Q_{air\ 1}$, $Q_{air\ 2}$ | Flow |
| $V$, $V_{Air\ 1}$, $V_{Air\ 2}$, $V_{Liquid\ 1}$, $V_{Liquid\ 2}$ | Volume |

**Claims**

1. A tanker comprising a detection device (2) for detection of the gas content of a liquid-containing fluid, **characterised in that** it comprises a gas separator (24) arranged to degas the liquid-containing fluid before the liquid-containing fluid enters the detection device (2), which detection device (2) comprises a pressure difference inducing member (28, 40), a first flow sensor (32) arranged at a first side of the pressure difference inducing member (28, 40) and a second flow sensor (32') arranged at the other side of the pressure difference inducing member (28, 40), a control unit (34) configured to determine the gas content of the liquid-containing fluid by means of the flow measurements made by the flow sensors (32, 32').

2. A tanker according to claim 1, **characterised in that** the detection device (2) comprises a first pressure sensor (30) arranged at a first side of the pressure difference inducing member (28, 40) and a second pressure sensor (30') arranged at the other side of the pressure difference inducing member (28, 40).

3. A tanker according to claim 1, **characterised in that** detection device (2) comprises one pressure sensor (30, 30') configured to detect the pressure difference across the pressure difference inducing member (28, 40).

4. A tanker according to one of the preceding claims, **characterised in that** the pressure difference inducing member (28, 40) is a valve (28) or a pump (40) or a pipe or a motor.

5. A tanker according to one of the preceding claims, **characterised in that** the sensors (30, 30', 32, 32') are configured to provide continuous monitoring measurements.

6. A tanker according to one of the claims 1-4, **characterised in that** the sensors (30, 30', 32, 32') are configured to provide intermittent measurements.

7. Method for detection of the gas content of a liquid-containing fluid in a tanker according to claims 1-6, **characterised in that** the method comprises the following steps:

   - passing the liquid-containing fluid through a gas separator (24) before the gas content of the liquid-containing fluid is detected;
   - establishing a pressure difference across a pressure difference inducing member (28, 40);
   - detecting a first flow ($Q_1$) at a first side of the pressure difference inducing member (28, 40) by means of a first flow sensor (32) arranged at the first side of the pressure difference inducing member (28, 40);
   - detecting a second flow ($Q_2$) at the other side of the pressure difference inducing member (28, 40) by means of a second flow sensor (32') arranged at the other side of the pressure difference inducing member (28, 40);
   - determining the gas content of the liquid-containing fluid by means of the flow measurements made by the flow sensors (32, 32').

8. Method according to claim 7, **characterised in that** the method comprises the step of measuring the pressure at each side of the pressure difference inducing member (28, 40) by means of one or more pressure sensors (30, 30').

9. Method according to claim 7 or claim 8, **characterised in that** the pressure difference inducing member (28, 40) is a valve (28) or a pump (40) or a pipe or a motor.

10. Method according to claim 9, **characterised in that** the pressure difference inducing member (28, 40) is a constant pressure valve.

11. Method according to one of the preceding claims 7-10, wherein a detection device (2) is applied.

**12.** A tanker according to one of the claims 1-6, **characterised in that** the pressure difference inducing member (28, 40) is formed as a spring-loaded check valve (28) arranged between a first pressure sensor (30) which is arranged at the low pressure side of the check valve (28) and a second flow sensor (32') which is arranged at the high pressure side of the check valve (28).

**13.** A tanker according to one of the claims 1-6 or claim 12, **characterised in that** the pressure difference inducing member (40) is configured to introduce a pressure drop causing the second pressure sensor (30') arranged at the low pressure side of the pressure difference inducing member (40) to detect a pressure ($P_2$) that is lower than the pressure ($P_1$) measured by means of the first pressure sensor (30).

**14.** A tanker according to one of the claims 1-6 or claims 12-13, **characterised in that** the tanker comprises a container (6) and an air evacuation pipe (8"") being in fluid communication with the container (6), preferably with the upper portion of the container (6) of the tanker, wherein the evacuation pipe (8"") is connected to the gas separator (24) through a connection pipe (8"').

**15.** A tanker according to one of the claims 1-6 or claims 12-14, **characterised in that** the detection device (2) comprises a gas separator (24) shaped as a tank provided with a dipstick (22) having a stick member (26).

Fig. 1

A)

B)

Fig. 2

Fig. 3

Low pressure side

$V_{Liquid\ 2}$

$V_{Air\ 2}$

44

$P_2$

10'

40

$\triangle P = P_1 - P_2$

$V_{Liquid\ 1}$

$V_{Air\ 1}$

10

42

$P_1$

High pressure side

# Fig. 4

Fig. 5

Fig. 6

Europäisches
Patentamt

European
Patent Office

Office européen
des brevets

# EUROPEAN SEARCH REPORT

**Application Number**

EP 16 15 2006

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 847 898 B1 (CHEN QINGYUAN [US] ET AL) 25 January 2005 (2005-01-25) | 1-13 | INV.<br>G01F1/74 |
| Y | * column 2, lines 9-43 *<br>* column 3, lines 28-46 *<br>* column 11, lines 20-37 *<br>* column 11, line 60 - column 12, line 6 *<br>* column 21, line 46 - column 22, line 32 *<br>* column 25, lines 13-33; figures 2,8 * | 14,15 | G01F15/08<br>B67D7/08<br>A01J5/01 |
| Y | GB 2 152 679 A (MILK MARKETING BOARD) 7 August 1985 (1985-08-07)<br>* abstract *<br>* page 2, lines 44-75; figure 1 * | 14 | |
| Y | DE 10 2009 041571 A1 (SCHWARTE JANSKY GMBH [DE]) 10 March 2011 (2011-03-10)<br>* paragraphs [0001], [0060]; figure 1 * | 15 | |
| Y | US 6 601 000 B1 (BARLIAN REINHOLD [DE] ET AL) 29 July 2003 (2003-07-29)<br>* column 1, lines 34-57 *<br>* column 6, lines 14-51; figure 2 * | 15 | TECHNICAL FIELDS SEARCHED (IPC)<br><br>A01J<br>B67D<br>G01F<br>G01N |

The present search report has been drawn up for all claims

| Place of search | Date of completion of the search | Examiner |
|---|---|---|
| The Hague | 10 June 2016 | Myrillas, K |

EPO FORM 1503 03.82 (P04C01)

## ANNEX TO THE EUROPEAN SEARCH REPORT
## ON EUROPEAN PATENT APPLICATION NO.

EP 16 15 2006

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2016

| Patent document cited in search report | | Publication date | Patent family member(s) | | Publication date |
|---|---|---|---|---|---|
| US 6847898 | B1 | 25-01-2005 | US | 6847898 B1 | 25-01-2005 |
| | | | US | 2005109078 A1 | 26-05-2005 |
| | | | WO | 2005024351 A2 | 17-03-2005 |
| GB 2152679 | A | 07-08-1985 | NONE | | |
| DE 102009041571 | A1 | 10-03-2011 | DE | 102009041571 A1 | 10-03-2011 |
| | | | DK | 2475244 T3 | 14-07-2014 |
| | | | EP | 2475244 A2 | 18-07-2012 |
| | | | WO | 2011029532 A2 | 17-03-2011 |
| US 6601000 | B1 | 29-07-2003 | AT | 235042 T | 15-04-2003 |
| | | | CA | 2340949 A1 | 09-03-2000 |
| | | | DE | 19839112 A1 | 09-03-2000 |
| | | | DE | 29825189 U1 | 08-12-2005 |
| | | | DE | 29825190 U1 | 08-12-2005 |
| | | | DE | 29825191 U1 | 08-12-2005 |
| | | | DE | 59904654 D1 | 24-04-2003 |
| | | | EP | 1105701 A1 | 13-06-2001 |
| | | | ES | 2195596 T3 | 01-12-2003 |
| | | | PL | 346278 A1 | 28-01-2002 |
| | | | PT | 1105701 E | 31-07-2003 |
| | | | US | 6601000 B1 | 29-07-2003 |
| | | | WO | 0012972 A1 | 09-03-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4168624 A
- US 4050896 A
- EP 1686355 A1
- WO 9115738 A1
- DE 102005005295 A1
- WO 2014021884 A1